# EUROPEAN PATENT SPECIFICATION

(11) **EP 1 716 842 B1**
(45) Date of publication and mention of the grant of the patent: **24.12.2008**
(21) Application number: 05009558.7
(22) Date of filing: 30.04.2005
(51) Int. Cl.: A61K 8/46, A61K 8/44, A61K 8/60, A61K 8/64, A61K 8/37, A61Q 5/00, A61Q 19/10

(54) **Mild cleansing compositions**
Milde Reinigungszusammensetzung
Composition de nettoyage douce

(43) Date of publication of application: 02.11.2006
(73) Proprietor: Cognis IP Management GmbH, 40589 Düsseldorf (DE)
(72) Inventor: Amela Conesa, Cristina, 08290 Cerdanyola del Vallés (ES); Prat Queralt, Esther, 08328 Alella (ES)
(74) Representative: Fabry, Bernd

(56) References cited:
- WO-A-01/85106
- WO-A-20/04078159
- DE-A1- 10 140 150

## Description

### Object of the invention

The present invention relates to mild cleansing, and more particular mild shampoo compositions, mainly for baby care, comprising a synergistic mixture of anionic and non-ionic surfactants and as well as the use of said mixture for making cosmetic compositions.

### State of the art

Mild shampoo compositions which provide low irritation to the skin are highly desirable. Conventional cleansings contain high levels of harsh anionic surfactants. These materials can penetrate the skin and destroy its integrity. This results, at the very least, in rough skin, and can ultimately lead to red, irritated skin. Ideal cleansing compositions should provide sufficient lathering and cleaning benefits to cleanse the hair while at the same time causing little or no irritation to the skin. This is particularly essential for cleansing compositions used on babies, small children, or adults with dry or sensitive skin. Furthermore, children can have difficulty in getting cleansing in their eyes during the hair washing or rinsing process. Hence, mild shampoo compositions which also provide reduced eye sting are particularly desirable for use on children.

In order to overcome the irritation problem for example, US 5,514,369 on behalf of Henkel Corp. suggests a cleansing formulation free of anionic surfactants, comprising a mixture of alkyl oligoglucosides, betaines and polymeric slip agents in order to provide a mild composition. Another example is EP-0548265-B1 on behalf of-Procter & Gamble which suggests a mild shampoo composition comprising an anionic surfactant, a combination of two amphoteric surfactants; the first being selected from certain imidazolinium derivatives, and the second being selected from aminoalkanoates and/or iminodialkanoates; and optionally, a non-ionic surfactant, to provide eye sting reduction benefits. Moreover, International patent application WO 04/073666 A1 of Beiersdorf claims a cosmetic and/or dermatological cleaning composition, comprising (a) one or more non-ionic surfactants chosen from the group consisting of decyl glycoside, lauryl glycoside and coco glycoside, (b) one or more anionic surfactants chosen from the group consisting of sodium cocoylglutamate, sodium lauryl ether sulfate, sodium sarcosinate, sodium myristyl ether sulfate, and (c) glyceryl oleate, and (d) optionally further cosmetic or dermatological auxiliary agents, under the proviso that the water content - calculated on the total composition - is at least 75 % b.w. and its viscosity is less than 1,000 mPas. The problem, however, which underlies this invention has been to provide a foaming composition with a viscosity which is low enough for spraying. The application neither claims nor suggests compositions having a specific mildness, especially for cleansinging of babies.

The international patent application WO01/85106 describes a baby shampoo which are mild to the skin which composition comprises 11 weight-% TEXAPON (TM) K14S, 5 % coco-glucoside and glyceryl oleate and 5 % cocamidopropylbetain.

The lathering skin-care product as described in WO2004/078159 contains 4 weight-% of Na-laureth sulfate, 5 % of coco glucoside, 0.14 % of PEG 150 distearate as well as 0.60 % of hydroxypropyltrimonium hydrolysed wheat protein.

The German patent specification discloses a skin- and hair-care cleansing composition comprising 8 weight-% of sodium laureth sulfate, 4 % of coco-glucoside, about 0.2 % of PEG 7 glyceryl cocoate or glyceryl oleate.

The formulation of such mild shampoo compositions, however, generally is an exercise in efficacy and mildness trade-offs, with the resulting composition providing either good cleaning, foaming and lathering benefits, or mildness and low eye or skin irritation benefits. In fact, Johnson's Baby Shampoo, while being particularly mild to the skin and eyes, is not a very good cleaner and its lathering profile is not particularly robust. Hence, it is an object of the present invention to provide a mild shampoo composition which provides good in-use characteristics such as foaming, lathering, both in terms of abundance of lather and lather stability, and cleaning, while at the same time providing skin mildness benefits, and preferably low eye sting benefits as well.

Also desirable in state of the art cleansing compositions are conditioning benefits inherent in the cleansing compositions. Such products are often termed two-in-one shampoos, meaning the composition includes both cleaning and conditioning ingredients in the same product. Such compositions are difficult to formulate because the cleaning ingredients, in general, tend to be incompatible with the conditioning ingredients. Hence, it is a further object of the present invention to provide a mild shampoo composition which provides excellent lathering and cleaning benefits while at the same time providing hair conditioning benefits through the use of suitable cationic conditioning agents.

### Description of the invention

The present invention claims mild shampoo compositions, comprising
(a) 1 to 9, preferably 2 to 7 % b.w. alkyl ether sulfates,
(b) 0.1 to 3 % b.w. N-acyl amino acids and/or alk(en)yl oligoglycoside carboxylates,
(c) 0.1 to 5 % b.w. alk(en)yl oligoglycosides,
(d) 0.1 to 2 % b.w. partial glycerides, and optionally
(e) 0.1 to 5 % b.w. amphoteric and/or zwitterionic surfactants and/or
(f) 0.1 to 1 % b.w. cationic proteins.
under the condition that the compounds add with water and optionally other typical auxiliary agents to 100 % b.w.

To his surprise, applicant has found that a composition consisting of compounds (a) to (d) in the given amounts provides a cleansing composition of high foaming, cleaning and lathering power (which is typical for anionic surfactants), without the usual disadvantage of insufficient mildness, especially irritation of eyes. In contrast, in the so-called HET-CAM test the compositions according to the present invention exhibit a synergistic effect with respect to mildness. The addition of amphoteric surfactant leads to a further improvement of foaming power and mildness, while cationic proteins have been found to be the preferred conditioners, since they are easy to be incorporated in the solution, do not alter the viscosity and do not separate neither during storage nor under the influence of temperature. The compositions exhibit a viscosity of 1.000 to 4000, preferably 1.200 to 3500 and most preferably 2000 to 3100 mPas (according to Brookfield RVT, spindle 2, 10 rpm, 20 °C), so that they are easy to handle and can be pumped out of a container.

### Alkyl ether sulfates

It is known that alkyl ether sulfates ("ether sulfates") which form component (a) are anionic surfactants which are industrially produced by the sulfation of fatty alcohol or oxoalcohol polyglycol ethers with SO₃ or chlorosulfonic acid (CSA) and subsequent neutralization. Alkyl ether sulfates suitable for the purposes of the invention correspond to formula **(I):**

**R¹(OCH₂CHR²)ₙOSO₃X** **(I)**

in which R¹ is a linear or branched alkyl and/or alkenyl group containing 12 to 18 carbon atoms, R² is hydrogen or methyl, n is a number of 1 to 8 and X is alkali, alkaline earth, ammonium, alkylammonium, alkanolammonium or glucammonium. Typical examples are the sulfates of addition products of on average 1 to 8 and, more particularly, 2 to 3 moles of ethylene oxide onto caproic alcohol, caprylic alcohol, 2-ethyl hexyl alcohol, capric alcohol, lauryl alcohol, isotridecyl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and brassidyl alcohol and technical mixtures thereof in the form of their alkyl, preferably monoisopropanolammonium salts. The ether sulfates may have both a conventional homologue distribution and a narrow homologue distribution. It is particularly preferred to use ether sulfates based on addition products of, on average, 2 to 3 moles of ethylene oxide with technical C₁₂-C₁₈, preferably C₁₂-C₁₄ fatty alcohol fractions preferably in the form of their sodium, magnesium, ammonium or monoisopropanolammonium salts.

### N-acylamino acids

Basically, the N-acylamino acids which form component (b1) may be derived from any α-amino acids which can be acylated with fatty acid halides to form N-acylamino acids. Preferred amino acids are glutamic acid, sarcosine, aspartic acid, alanine, valine, leucine, isoleucine, proline, hydroxyproline, lysine, glycine, serine, cystein, cystine, threonine, histidine and salts thereof and, more particularly, glutamic acid, sarcosine, aspartic acid, glycine, lysine and salts thereof. The amino acids may be used in optically pure form or as racemic mixtures. The amino acid components of the N-acylamino acids are preferably derived from glutamic acid and/or aspartic acid, i.e. N-acyl glutamates and N-acyl aspartates are preferably used. In addition, the acyl groups of the N-acylamino acids may be derived from fatty acids corresponding to formula (II):

**R³CO-OH** **(II)**

in which R³ is a linear or branched acyl group containing 6 to 22 carbon atoms and 0 and/or 1 to 3 double bonds. Typical examples are acyl groups derived from caproic acid, caprylic acid, capric acid, lauric acid, mrystic acid, palmitic aid, stearic acid, isostearic acid, oleic acid, elaidic acid, linoleic acid, linolenic acid, gadoleic acid, arachidonic acid, behenic acid and erucic acid and technical mixtures thereof. The N-acylamino acids are preferably derived from technical C₁₂₋₁₈ coconut oil fatty acids. The N-acylamino acids may be present in acidic form, but are generally used in the form of their salts, preferably alkali metal or ammonium salts.

The sodium and triethanolamine salts are particularly preferred. Overall, N-cocoyl glutamate is the preferred N-acylamino acid.

### Alk(en)yl oligoglycoside carboxylates

The alk(en)yl oligoglycoside carboxylates which can be used in the compositions according to the invention as component (b2) correspond to formula **(III)** :

**R⁴O[G]ₚO[(CH₂)ₘCOO⁻X⁺]ₙ** **(III)**

wherein R⁴ is an alkyl or alkenyl radical having from 6 to 22 carbon atoms, G is a sugar unit having 5 or 6 carbon atoms, p is a number from 1 to 10, m and n are numbers from 1 to 5, and X is alkali, alkaline earth, ammonium, alkylammonium, alkanolammonium or glucammonium. The products are obtainable according to the methods known from the art, for example by reaction of alk(en)yl oligoglycosides with halogen carboxylic acids or their salts in alkaline medium an in the presence of solvents. The alkyl or alkenyl oligoglycosides carboxylates may be derived from aldoses or ketoses containing 5 or 6 carbon atoms, preferably glucose. Accordingly, the preferred alkyl and/or alkenyl oligoglycoside carboxylates are alkyl or alkenyl oligo**glucoside** carboxylates. The index p in general formula **(III)** indicates the degree of oligomerisation (DP degree), i.e. the distribution of mono- and oligoglycosides, and is a number of 1 to 10. Whereas p in a given compound must always be an integer and, above all, may assume a value of 1 to 6, the value p for a certain alkyl oligoglycoside moiety is an analytically determined calculated quantity which is mostly a broken number. Alk(en)yl oligoglycoside carboxylates having an average degree of oligomerisation p of 1.1 to 3.0 are preferably used. Alk(en)yl oligoglycoside carboxylates having a degree of oligomerisation below 1.7 and, more particularly, between 1.2 and 1.4 are preferred from the applicational point of view.

The alkyl or alkenyl radical R⁴ may be derived from primary alcohols containing 4 to 22 and preferably 8 to 16 carbon atoms. Typical examples are butanol, caproic alcohol, caprylic alcohol, capric alcohol, undecyl alcohol, lauryl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and technical mixtures thereof such as are formed, for example, in the hydrogenation of technical fatty acid methyl esters or in the hydrogenation of aldehydes from Roelen's oxo synthesis. Alkyl oligoglucosides based on hydrogenated C₈-C₁₆ coconut oil alcohol having a DP of 1 to 3 are preferred.

Furtheron, the alk(en)yl oligoglycoside carboxylates may be derived from carboxylic acids, their salts or esters, in which the acyl moiety comprises 1 to 5, preferably 2 to 4 and more preferably 1 or 2 carbon atoms, while the number of acyl groups in the alk(en)yl oligoglycoside carboxylate may be 1 to 5 and preferably 1 to 3. Moreover, X stands preferably for potassium, ammonium, triethanolammonium and most preferably for sodium.

The carboxylic acids which can be used for preparing the alk(en)yl oligoglycoside carboxylates usually comprise 1 to 4 carbon atoms; preferably acetic acid, its esters or its salts, particularly its sodium or potassium salt are used. In a preferred embodiment of the present invention alk(en)yl oligoglycoside carboxylates are used which are obtained by reaction of an aqueous solution of an alk(en)yl oligoglycoside, having e.g. 20 to 70 % b.w. solids matter, under nitrogen and in presence of an alkaline catalyst, e.g. alkali hydroxide or alkali carbonate, at a temperature of from 50 to 100 °C with ω-halogen carboxylic acid, its ester or salt, like for example potassium or sodium monocloroacetate in a molar ratio of from 1 : 0.5 to 1 : 5, preferably 1 : 1 to 1 : 3. The molar ratio of alkali : halogen carboxylic acid, its esters or salts is usually adjusted to 1 : 0.5 to 1 : 1.5 and preferably 1 : 1.1. The preparation of the medium chain C_{12/14} alkyl oligoglycoside carboxylates usually takes place in the absence of organic solvents, while the long chain C_{16/18} alkyl oligoglycoside carboxylates are prepared in the presence of long chain fatty alcohols or 1,2-propylene glycol.

### Alk(en)yl oligoglycosides

The alkyl or alkenyl oligoglycosides which can be used in the compositions according to the invention as component (c) may be derived from aldoses or ketoses containing 5 or 6 carbon atoms, preferably glucose. Accordingly, the preferred alkyl and/or alkenyl oligoglycosides are alkyl or alkenyl oligoglucosides. These materials are also known generically as "alkyl polyglycosides" (APG). The alk(en)yl oligoglycosides according to the invention correspond to formula **(IV) :**

**R⁵O[G]ₚ** **(IV)**

wherein R⁵ is an alkyl or alkenyl radical having from 6 to 22 carbon atoms, G is a sugar unit having 5 or 6 carbon atoms and p is a number from 1 to 10. The index p in general formula **(IV)** indicates the degree of oligomerisation (DP degree), i.e. the distribution of mono- and oligoglycosides, and is a number of 1 to 10. Whereas p in a given compound must always be an integer and, above all, may assume a value of 1 to 6, the value p for a certain alkyl oligoglycoside is an analytically determined calculated quantity which is mostly a broken number. Alk(en)yl oligoglycosides having an average degree of oligomerisation p of 1.1 to 3.0 are preferably used. Alk(en)yl oligoglycosides having a degree of oligomerisation below 1.7 and, more particularly, between 1.2 and 1.4 are preferred from the applicational point of view.

The alkyl or alkenyl radical R⁵ may be derived from primary alcohols containing 4 to 22 and preferably 8 to 16 carbon atoms. Typical examples are butanol, caproic alcohol, caprylic alcohol, capric alcohol, undecyl alcohol, lauryl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and technical mixtures thereof such as are formed, for example, in the hydrogenation of technical fatty acid methyl esters or in the hydrogenation of aldehydes from Roelen's oxo synthesis. Alkyl oligoglucosides based on hydrogenated C₈-C₁₆ coconut oil alcohol having a DP of 1 to 3 are preferred.

### Partial glycerides

Partial glycerides, i.e. monoglycerides, diglycerides and technical mixtures thereof, which form component (d), may still contain small quantities of triglycerides from their production. The partial glycerides preferably correspond to formula (V): 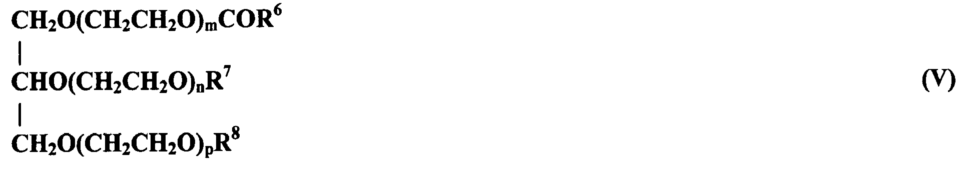 in which R⁶CO is a linear or branched, saturated and/or unsaturated acyl group containing 6 to 22 and preferably 12 to 18 carbon atoms, R⁷ and R⁸ independently of one another have the same meaning as R⁶CO or represent a hydroxyl radical and the sum (m+ n+p) is 0 or a number of 1 to 100 and preferably 5 to 25, with the proviso that at least one of the two substituents R⁷ and R⁸ is a hydroxyl radical. Typical examples are mono and/or diglycerides based on caproic acid, caprylic acid, 2-ethylhexanoic acid, capric acid, lauric acid, isotridecanoic acid, myristic acid, palmitic acid, palmitoleic acid, stearic acid, isostearic acid, oleic acid, elaidic acid, pet-roselic acid, linoleic acid, linolenic acid, elaeostearic acid, arachic acid, gadoleic acid, behenic acid and erucic acid and technical mixtures thereof. Technical lauric acid glycerides, palmitic acid glycerides, stearic acid glycerides, isostearic acid glycerides, oleic acid glycerides, behenic acid glycerides and/or erucic acid glycerides which have a monoglyceride content of 50 to 95% by weight and preferably 60 to 90% by weight are preferably used.

### Amphoteric and zwitterionic surfactants

Amphoteric and/or zwitterionic surfactants, which form the optional component (e), can be divided into several groups, like alkyl betaines, alkylamido betaines, imidazolins and amphoglycinates.
- Alkyl betaines
   The betaines are known surfactants which are mainly produced by carboxyalkylation, preferably carboxymethylation, of amine compounds. The starting materials are preferably condensed with halocarboxylic acids or salts thereof, more particularly sodium chloroacetate, one mole of salt being formed per mole of betaine. The addition of unsaturated carboxylic acids, such as acrylic acid for example, is also possible. Examples of suitable betaines are the carboxyalkylation products of secondary and, in particular, tertiary amines which correspond to formula (VI): 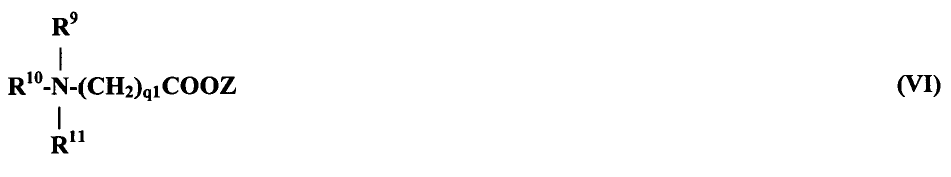 where R⁹ is a an alkyl radical having 6 to 22 carbon atoms, R¹⁰ is hydrogen or an alkyl group containing 1 to 4 carbon atoms, R¹¹ is an alkyl group containing 1 to 4 carbon atoms, q1 is a number of 1 to 6 and X is an alkali and/or alkaline earth metal or ammonium. Typical examples are the carboxymethylation products of hexylmethylamine, hexyldimethylamine, octyldimethylamine, decyldimethylamine, C_{12/14}-cocoalkyldimethylamine, myristyldimethylamine, cetyldimethylamine, stearyldimethylamine, stearylethylmethylamine, oleyldimethylamine, C_{16/18}-tallowalkyldimethylamine and their technical mixtures, and particularly dodecyl methylamine, dodecyl dimethylamine, dodecyl ethylmethylamine and technical mixtures thereof. The commercially available products include Dehyton^{®} AB (Cognis Deutschland GmbH & Co., KG)
- Alkylamido betaines
   Other suitable betaines are the carboxyalkylation products of amidoamines which correspond to formula **(VII):** 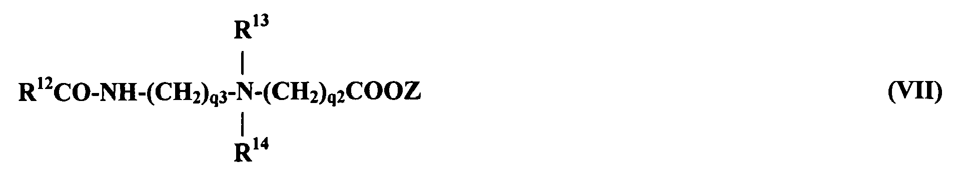 in which R¹²CO is an aliphatic acyl radical having 6 to 22 carbon atoms and 0 or 1 to 3 double bonds, R¹³ is hydrogen or an alkyl radical having 1 to 4 carbon atoms, R¹⁴ is an alkyl radical having 1 to 4 carbon atoms, q2 is a number from 1 to 6, q3 is a number from 1 to 3 and Z is an alkali and/or alkaline earth metal or ammonium. Typical examples are reaction products of fatty acids having 6 to 22 carbon atoms, like for example caproic acid, caprylic acid, caprinic acid, lauric acid, myristic acid, palmitic acid, palmoleic acid, stearic acid, isostearic acid, oleic acid, elaidic acid, petroselinic acid, linolic acid linoleic acid, elaeostearic acid, arachidonic acid, gadoleic acid, behenic acid, erucic acid and their technical mixtures with N,N-dimethylaminoethylamine, N,N-dimethylaminopropylamine, N,N-diethylaminoethylamine und N,N-diethylaminopropylamine, which are condensed with sodium chloroacetate. The commercially available products include Dehyton^{®} K and Dehyton^{®} PK (Cognis Deutschland GmbH & Co., KG) as well as Tego^{®}Betaine (Gold-schmidt).
- Imidazolines
   Other suitable starting materials for the betaines to be used for the purposes of the invention are imidazolines. These substances are also known and may be obtained, for example, by cyclizing condensation of 1 or 2 moles of C₆-C₂₂ fatty acids with polyfunctional amines, such as for example aminoethyl ethanolamine (AEEA) or diethylenetriamine. The corresponding carboxyalkylation products are mixtures of different open-chain betaines. Typical examples are condensation products of the above- mentioned fatty acids with AEEA, preferably imidazolines based on lauric acid, which are subsequently betainised with sodium chloroacetate. The commercially available products include Dehyton® G, Dehyton^{®} MC, Dehyton^{®} DC (all Cognis Deutschland GmbH & Co., KG)

### Cationic proteins

Cationic proteins, forming optional component (f) according to the present invention, which have been prepared by enzymatic methods are preferably used for the purposes of the invention. Enzymes acting specifically on the peptide bond are used in these methods. The products have a high homogeneity and, in particular, show improved dermatological compatibility in relation to the other hydrolysates. Applicants have found that vegetable products based on degradation products of pea, rice, almond, potato, soy, silk and, in particular, wheat proteins (for example gluten) are particularly suitable for this purpose. The acidic, alkaline and/or enzymatic hydrolysis of these starting materials generally leads to products which have an average molecular weight in the range from 100 to 10,000, preferably in the range from 100 to 5,000 and more particularly in the range from 200 to 1,000 Dalton.

For the hydrolysis, an aqueous suspension of the protein-containing starting material is typically degraded - optionally together with the adsorbents, such as active carbon for example - over a period of 1 to 24 h at varying pH values and at the optimum temperature of the proteinases and peptidases used below 70°C and preferably below 60°C, for example at 30 to 55°C. After the hydrolysis, it is advisable to adjust the pH into the acidic range, for example to pH 3 to 4. If the hydrolysis is carried out in the presence of calcium oxide or calcium hydroxide as base, calcium peptides are formed and have to be filtered off from the residue. If the alkali metal peptides are required, it is advisable to treat the calcium peptides with soda or potash solution and then to remove the poorly soluble calcium carbonate. The calcium may also be precipitated in the form of calcium sulfate or calcium oxalate. The poorly soluble salts are preferably separated off in the presence of filter aids through nutsch filters or filter presses. Aqueous vegetable protein hydrolysate solutions are obtained and, if desired, may be concentrated, for example, using falling film evaporators. The hydrolysates obtainable in this way generally have a solids content of about 5 to 50% by weight.

The cationisation of the vegetable protein hydrolysates thus obtained takes place between the free amino and/or carboxyl groups of the oligopeptide and a halogen atom of the quaternary ammonium salt used, hydrogen halide being given off. The quaternary ammonium salts preferably used correspond to formula **(VIII):** 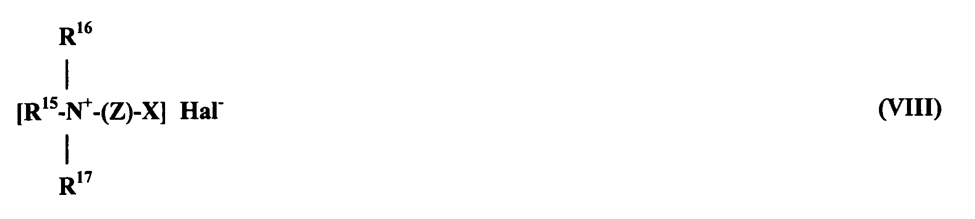 in which R¹⁵ is an alkyl and/or alkenyl group containing 1 to 22 carbon atoms, R¹⁶ and R¹⁷ independently of one another represent an alkyl group containing 1 to 4 carbon atoms, Z is an optionally hydroxysubstituted alkylene group and X and Hal independently of one another represent chlorine or bromine. In one particular embodiment of the invention, the quaternary ammonium salts used are N,N-dimethyl-N-(n-alkyl)-N-(2-hydroxy-3-chloro-n-propyl)-ammonium halides and, in particular, N,N-dimethyl-N-(n-dodecyl)-N-(2-hydroxy-3-chloro-n-propyl)-ammonium chloride.

As already mentioned, the reaction between the protein hydrolysates and the quaternary ammonium salts is accompanied by the elimination of hydrogen halide and is catalysed by alkali metal bases. Basically, the choice of the base is not critical although concentrated aqueous solutions of sodium or potassium hydroxide are preferably used. Accordingly, the pH value during the reaction is in the range from 8 to 12 and more particularly around 10. It has proved to be of advantage to select the molar ratios so that, for every mol of a protein hydrolysate which contains on average p mol peptide units, there are p/10 to p/100 mol and preferably p/20 to p/50 mol quaternary ammonium salts. In other words, this means that, per mol of protein hydrolysate containing on average 100 peptide units (p = 100), 1 to 10 mol (p/100 to p/10) and preferably 2 to 5 mol (p/50 to p/20) quaternary ammonium salts are used.

The reaction normally takes place at a temperature in the range from 20 to 90°C and preferably at a temperature in the range from 40 to 60°C. The reaction time is typically 1 to 24 h and, more particularly, 4 to 12 h. It has proved to be of advantage to adjust the end product to a neutral pH by addition of mineral acid and to stabilize it against microbial infestation in the usual way, i.e. for example by addition of PHB esters. The commercially available products include Gluadin^{®} WQ (Cognis Deutschland GmbH & Co. KG, Düsseldorf). Low molecular weight protein hydrolysates thus have such a small molecule size that these "microproteins" are even capable e.g. of penetrating into the textile fibres and repairing, strengthening and protecting them. So far as the amino acid composition of the quaternised protein hydrolysates according to the invention is concerned, the high glutamic acid content is particularly emphasized. Glutamic acid generally occurs widely in nature and can therefore be found in almost all proteins. However, the highest concentration occurs in wheat protein which generally contains more than 30% glutamic acid. Accordingly, it is from gluten, the protein of wheat gluten from which glutamic acid was first obtained, that the name of this protein unit derives. Although glutamic acid is not an essential amino acid, it does play an important role in various metabolism processes.

### Industrial application

Another object of the present invention is the use of mixtures comprising
(a) 1 to 9 % b.w. alkyl ether sulfates,
(b) 0.1 to 3 % b.w. N-acyl amino acids and/or alk(en)yl oligoglycoside carboxylates,
(c) 0.1 to 5 % b.w. alk(en)yl oligoglycosides,
(d) 0.1 to 2 % b.w. partial glycerides, and optionally
(e) 0.1 to 5 % b.w. amphoteric and/or zwitterionic surfactants and/or
(f) 0.1 to 1 % b.w. cationic proteins.
under the condition that the compounds add with water agents to 100 % b.w. for making cosmetic compositions, more particular, cleansing compositions.

### Cosmetic compositions

The preparations according to the invention may contain oil bodies, emulsifiers, superfatting agents, pearlising waxes, consistency factors, polymers, silicone compounds, waxes, stabilizers, primary and secondary sun protection agents, antidandruff agents, biogenic agents, film formers, swelling agents, hydrotropes, preservatives, solubilizers, complexing agents, reducing agents, alkalising agents, perfume oils, dyes and the like as additional auxiliaries and additives.

### Oil bodies

Suitable oil bodies, which form constituents of the O/W emulsions, are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acid with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆ -C₁₀-fatty acids, liquid mono-/di-/triglyceride mixtures based on C₆-C₁₈-fatty acids, esters of C₆- C₂₂-fatty alcohols and/or Guerbet alcohols with aromatic carboxylic acids, in particular benzoic acid, esters of C₂- C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol® CC), Guerbet carbonates, based on fatty alcohols having 6 to 18, preferably 8 to 10, carbon atoms, esters of benzoic acid with linear and/or branched C₆-C₂₂-alcohols (e.g. Finsolv® TN), linear or branched, symmetrical or asymmetrical dialkyl ethers having 6 to 22 carbon atoms per alkyl group, such as, for example, dicaprylyl ether (Cetiol® OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes. Emulsifiers

Other surfactants may also be added to the preparations as emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.
   The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:
- Partial glycerides
   Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.
- Sorbitan esters
   Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.
- Polyglycerol esters
   Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan® PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor® GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Typical anionic emulsifiers are aliphatic C₁₂₋₂₂ fatty acids, such as palmitic acid, stearic acid or behenic acid for example, and C₁₂₋₂₂ dicarboxylic acids, such as azelaic acid or sebacic acid for example.

### Superfatting agents

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

### Consistency factors

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents

Suitable thickeners are polymeric thickeners, such as Aerosil® types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400®, cationic starch, copolymers of diallyl ammonium salts and acrylamides, quaternized vinyl pyrrolidone/vinyl imidazole polymers such as, for example, Luviquat® (BASF), condensation products of polyglycols and amines, quaternized collagen polypeptides such as, for example, Lauryldimonium Hydroxypropyl Hydrolyzed Collagen (Lamequat® L, Grünau), quaternized wheat polypeptides, polyethyleneimine, cationic silicone polymers such as, for example, amodimethicone, copolymers of adipic acid and dimethylaminohydroxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®} A-15, Mirapol^{®} AD-1, Mirapol^{®} AZ-1 of Miranol.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Pearlising waxes

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates.

### Waxes

Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

### Stabilizers

Metal salts of fatty acids such as, for example, magnesium, aluminium and/or zinc stearate or ricinoleate may be used as stabilizers.

### Primary sun protection factors

Primary sun protection factors in the context of the invention are, for example, organic substances (light filters) which are liquid or cristalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances:
- 3-benzylidene camphor or 3-benzylidene norcamphor and derivatives thereof, for example 3-(4-methylbenzylidene)-camphor;
- 4-aminobenzoic acid derivatives, preferably 4-(dimethylamino)benzoic acid-2-ethylhexyl ester, 4-(dimethylamino)-benzoic acid-2-octyl ester and 4-(dimethylamino)benzoic acid amyl ester;
- esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene);
- esters of salicylic acid, preferably salicylic acid-2-ethylhexyl ester, salicylic acid-4-isopropylbenzyl ester, salicylic acid homomenthyl ester;
- derivatives of benzophenone, preferably 2-hydroxy-4-methoxybenzophenone, 2-hydroxy-4-methoxy-4'-methylbenzophenone, 2,2'-dihydroxy-4-methoxybenzophenone;
- esters of benzalmalonic acid, preferably 4-methoxybenzalmalonic acid di-2-ethylhexyl ester;
- triazine derivatives such as, for example, 2,4,6-trianilino-(p-carbo-2'-ethyl-1'-hexyloxy)-1,3,5-triazine and Octyl Triazone or Dioctyl Butamido Triazone (Uvasorb^{®} HEB);
- propane-1,3-diones such as, for example, 1-(4-tert.butylphenyl)-3-(4'-methoxyphenyl)-propane-1,3-dione;
- ketotricyclo(5.2.1.0)decane derivatives.

Suitable water-soluble substances are
- 2-phenylbenzimidazole-5-sulfonic acid and alkali metal, alkaline earth metal, ammonium, alkylammonium, alkanolammonium and glucammonium salts thereof;
- sulfonic acid derivatives of benzophenones, preferably 2-hydroxy-4-methoxybenzophenone-5-sulfonic acid and salts thereof;
- sulfonic acid derivatives of 3-benzylidene camphor such as, for example, 4-(2-oxo-3-bornylidenemethyl)-benzene sulfonic acid and 2-methyl-5-(2-oxo-3-bornylidene)-sulfonic acid and salts thereof.

Typical UV-A filters are, in particular, derivatives of benzoyl methane such as, for example, 1-(4'-tert.butylphenyl)-3-(4'-methoxyphenyl)-propane-1,3-dione, 4-tert.butyl-4-'methoxydibenzoyl methane (Parsol^{®} 1789) or 1-phenyl-3-(4'-isopropylphenyl)-propane-1,3-dione and the enamine compounds (BASF). The UV-A and UV-B filters may of course also be used in the form of mixtures. Particularly favourable combinations consist of the derivatives of benzoyl methane, for example 4-tert.butyl-4'-methoxydibenzoyl methane (Parsol^{®} 1789) and 2-cyano-3,3-phenylcinnamic acid-2-ethylhexyl ester (Octocrylene^{®}), in combination with esters of cinnamic acid, preferably 4-methoxycinnamic acid-2-ethylhexyl ester and/or 4-methoxycinnamic acid propyl ester and/or 4-methoxycinnamic acid isoamyl ester. Combinations such as these are advantageously combined with water-soluble filters such as, for example, 2-phenylbenzimidazole-5-sulfonic acid and alkali metal, alkaline earth metal, ammonium, alkylammonium, alkanolammonium and glucammonium salts thereof.

### Secondary sun protection factors

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

### Biogenic agents

In the context of the invention, biogenic agents are, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, for example prune extract, bambara nut extract, and vitamin complexes.

### Anti-microbial agents

Suitable anti-microbial agents are, in principle, all substances effective against Gram-positive bacteria, such as, for example, 4- hydroxybenzoic acid and its salts and esters, N-(4-chlorophenyl)-N'-(3,4- dichlorophenyl)urea, 2,4,4'-trichloro-2'-hydroxy-diphenyl ether (tri-closan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n- decylsalicylamide.

### Enzyme inhibitors

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

### Odour absorbers

Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odour-neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linaool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β- damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

### Antiperspirant active ingredients

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2- propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

### Film formers

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

### Antidandruff agents

Suitable antidandruff agents are Pirocton Olamin (1-hydroxy-4-methyl-6-(2,4,4-trimethylpentyl)-2-(1H)-pyridinone monoethanolamine salt), Baypival^{®} (Climbazole), Ketoconazol® (4-acetyl-1-{4-[2-(2,4-dichlorophenyl) r-2-(1H-imidazol-1-ylmethyl)-1,3-dioxylan-c-4-yl-methoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon^{®} UD (protein/undecylenic acid condensate), zinc pyrithione, aluminium pyrithione and magnesium pyrithione/dipyrithione magnesium sulfate.

### Hydrotropes

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Complexing agents

The complexing agents used may be selected from EDTA, NTA, phosphonic acids, Triton B, turpinal and phenacetin. In addition, reducing agents such as, for example, ascorbic acid, sodium sulfate, sodium thiosulfate and the like may be present. Suitable alkalizing agents are ammonia, monoethanolamines, (L) arginine, AMP, etc.

### Perfume oils

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, □-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### Dyes

Suitable dyes are any of the substances suitable and approved for cosmetic purposes as listed, for example, in the publication "**Kosmetische Färbemittel**" of the Farbstoffkommission der Deutschen Forschungsgemeinschaft, **Verlag Chemie, Weinheim, 1984, pages 81 to 106**. Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. These dyes are normally used in concentrations of 0.001 to 0.1 % by weight, based on the mixture as a whole.

The total percentage content of auxiliaries and additives may be from 1 to 50% by weight and is preferably from 5 to 40% by weight, based on the particular composition. The compositions may be produced by standard hot or cold processes.

### Examples

The following Table 1 provides a couple of formulation examples for the concept of mild shampoo compositions.

**Table 1**

| **Formulation examples (amounts in % b.w.)** | | | | | | |
|---|---|---|---|---|---|---|
| **Composition** | **1** | **2** | **3** | **4** | **5** | **6** |
| **Texapon^{®} NSO** | 7.0 | 7.0 | 13.0 | 6.0 | 6.0 | 6.0 |
| Sodium Laureth Sulfate | | | | | | |
| **Texapon^{®} ASV-50** | 3.8 | 3.8 | 3.0 | 6.0 | 6.0 | 6.0 |
| Sodium Laureth Sulfate (and) Sodium Laureth 8-Sulfate (and) Magnesium Laureth Sulfate (and) Mag-nesium Laureth 8-Sulfate (and) Sodium Oleth Sulfate (and) Magnesium Oleth Sulfate | | | | | | |
| **Plantapon^{®} ACG 35** | 1.0 | - | - | 3.5 | 3.5 | 3.5 |
| Disodium Cocoyl Glutamate | | | | | | |
| **Plantapon^{®}LCG Sorb** | - | 1.0 | 4.0 | - | - | - |
| Sodium Lauryl Glucose Carboxylate (and) Lauryl Glucoside | | | | | | |
| **Eumulgin^{®}EO-33** | 0.8 | 0.8 | - | - | 0.5 | 0.5 |
| PEG-150 Distearate | | | | | | |
| **Arlypon^{®} F** | - | - | - | 0.5 | - | - |
| Laureth-2 | | | | | | |
| **Cetiol^{®} LDO** | - | - | - | - | - | 0.2 |
| Dicaprylyl Ether (and) Lauryl Alcohol | | | | | | |
| **Dehyton^{®} MC** | 4.0 | 4.0 | 2.5 | 4.0 | 4.0 | 4.0 |
| Sodium Cocoamphoacetate | | | | | | |
| **Dehyton^{®} PK 45** | - | - | - | 3.1 | 3.1 | 3.1 |
| Cocamidopropyl Betaine | | | | | | |
| **Plantacare^{®} 2000** | - | - | - | - | 1.0 | - |
| Decyl Glucoside | | | | | | |
| **Lamesoft^{®} PO 65** | 1.0 | 1.0 | 3.0 | 1.5 | 1.5 | 1.5 |
| Coco-Glucoside (and) Glyceryl Oleate | | | | | | |
| **Gluadin^{®} WQ** | 1.0 | 1.0 | 1.0 | 1.5 | 1.5 | 1.5 |
| Laurdimonium Hydroxypropyl Hydrolyzed Wheat Protein | | | | | | |
| **NaCl** | q.s. | q.s. | q.s. | q.s. | q.s. | q.s. |
| Sodium Chloride | | | | | | |
| Water, preservatives | Up to 100 | Up to 100 | Up to 100 | Up to 100 | Up to 100 | Up to 100 |
| Viscosity [mPas]* | 1.600 | 1.600 | 2000 | 2800 | 3000 | 3100 |

| | | | | | | |
|---|---|---|---|---|---|---|
| *) Brookfield, RVT, Spindle 2, 10 rpm, 20 °C | | | | | | |

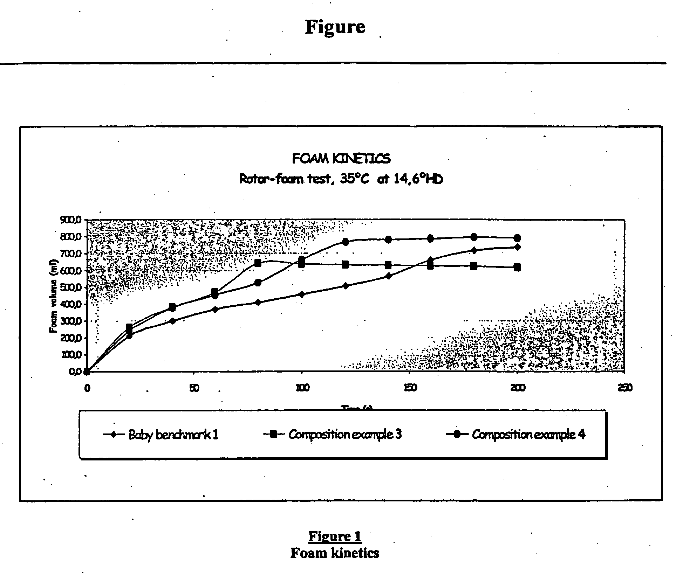

Foam kinetics versus Benchmark 1 (J&J Baby Shampoo) are given in Figure 1.

## Claims

1. Mild cleansing compositions, comprising
(a) 1 to 9 % b.w. alkyl ether sulfates,
(b) 0.1 to 3 % b.w. N-acyl amino acids and/or alk(en)yl oligoglycoside carboxylates,
(c) 0.1 to 5 % b.w. alk(en)yl oligoglycosides,
(d) 0.1 to 2 % b.w. partial glycerides, and optionally
(e) 0.1 to 5 % b.w. amphoteric and/or zwitterionic surfactants and/or
(f) 0.1 to 1% b.w. cationic proteins
under the condition that the compounds add with water and optionally other typical auxiliary agents to 100 % b.w.

2. Compositions according to claim 1, **characterized in that** said alkyl ether sulfates (compound a) correspond to formula **(I):**
**R¹(OCH₂CHR²)ₙOSO₃X** **(I)**
in which R¹ is a linear or branched alkyl and/or alkenyl group containing 12 to 18 carbon atoms, R² is hydrogen or methyl, n is a number of 1 to 8 and X is alkali, alkaline earth, ammonium, alkylammonium, alkanolammonium or glucammonium.

3. Compositions according to claims 1 and/or 2, **characterised in that** said N-acyl aminoacid (compound b1) is N-cocoyl glutamate.

4. Compositions according to any of claims 1 to 3, **characterised in that** said alk(en)yl oligoglycoside carboxylates (compound b2) correspond to formula **(III):**
**R⁴O[G]ₚO[(CH₂)ₘCOO⁻X⁺]ₙ** **(III)**
wherein R⁴ is an alkyl or alkenyl radical having from 6 to 22 carbon atoms, G is a sugar unit having 5 or 6 carbon atoms, p is a number from 1 to 10, m and n are numbers from 1 to 5, and X is alkali, alkaline earth, ammonium, alkylammonium, alkanolammonium or glucammonium.

5. Compositions according to any of claims 1 to 4, **characterised in that** said alk(en)yl oligoglycosides (compound c) corresponds to formula **(IV) :**
**R⁵O[G]ₚ** **(IV)**
wherein R⁵ is an alkyl or alkenyl radical having from 4 to 22 carbon atoms, G is a sugar unit having 5 or 6 carbon atoms and p is a number from 1 to 10.

6. Compositions according to any of claims 1 to 5, **characterised in that** said partial glycerides correspond to general formula **(V),** 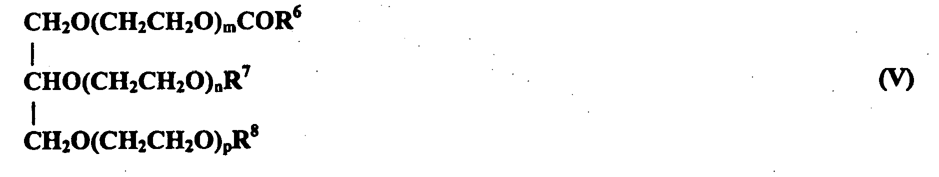 in which R⁶CO is a linear or branched, saturated and/or unsaturated acyl group containing 6 to 22 carbon atoms, R⁷ and R⁸ independently of one another have the same meaning as R⁶CO or represent a hydroxyl radical and the sum (m+ n+p) is 0 or a number of 1 to 100 a, with the proviso that at least one of the two substituents R⁷ and R⁸ is a hydroxyl radical.

7. Compositions according to any of claims 1 to 6, **characterised in that** said amphoteric and/or zwitterionic surfactants (compound e) are selected from the group consisting of alkyl betaines, alkylamido betaines, imidazolins and amphoglycinates.

8. Compositions according to claim 7, **characterised in that** said amphoteric and/or zwitterionic surfactants correspond to formula **(VI):** 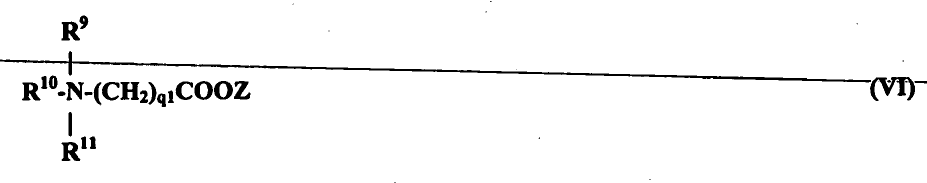 where R⁹ is a an alkyl radical having 6 to 22 carbon atoms, R¹⁰ is hydrogen or an alkyl group containing 1 to 4 carbon atoms, R¹¹ is an alkyl group containing 1 to 4 carbon atoms, q1 is a number of 1 to 6 and X is an alkali and/or alkaline earth metal or ammonium.

9. Compositions according to claim 7, **characterised in that** said amphoteric and/or zwitterionic surfactants correspond to formula **(VII):** 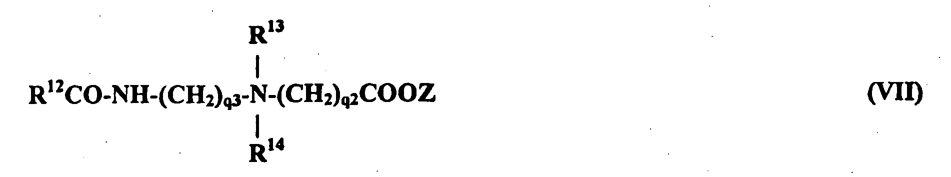 in which R¹²CO is an aliphatic acyl radical having 6 to 22 carbon atoms and 0 or 1 to 3 double bonds, R¹³ is hydrogen or an alkyl radical having 1 to 4 carbon atoms, R¹⁴ is an alkyl radical having 1 to 4 carbon atoms, q2 is a number from 1 to 6, q3 is a number from 1 to 3 and Z is an alkali and/or alkaline earth metal or ammonium.

10. Compositions according to any of the claims 1 to 9, **characterised in that** said cationic proteins (compound f) are based on degradation products of pea, rice, almond, potato, soy, silk and, in particular, wheat proteins.

11. Compositions according to any of the claims 1 to 10, **characterised in that** they exhibit a viscosity of 1,000 to 4,000 mPas.

12. Use of mixtures comprising
(a) 1 to 9 % b.w. alkyl ether sulfates,
(b) 0.1 to 3 % b.w. N-acyl amino acids and/or alk(en)yl oligoglycoside carboxylates,
(c) 0.1 to 5 % b.w. alk(en)yl oligoglycosides,
(d) 0.1 to 2% b.w. partial glycerides, and optionally
(e) 0.1 to 5 % b.w. amphoteric and/or zwitterionic surfactants and/or
(f) 0.1 to 1 % b.w. cationic proteins
under the condition that the compounds add with water to 100 % b.w. for making cosmetic compositions.

## Patentansprüche

1. Milde Reinigungzusammensetzungen, umfassend
(a) 1 bis 9 Gew.-% Alkylethersulfate,
(b) 0,1 bis 3 Gew.-% N-Acylaminosäuren und/oder Alk(en)yloligoglycosidcarboxylate,
(c) 0,1 bis 5 Gew.-% Alk(en)yloligoglycoside,
(d) 0,1 bis 2 Gew.-% Partialglyceride sowie gegebenenfalls
(e) 0,1 bis 5 Gew.-% amphotere und/oder zwitterionische Tenside und/oder
(f) 0,1 bis 1 Gew.-% kationische Proteine,
unter der Bedingung, daß die Komponenten mit Wasser und gegebenenfalls anderen typischen Hilfsmitteln 100 Gew.-% ausmachen.

2. Zusammensetzungen nach Anspruch 1, **dadurch gekennzeichnet, daß** die Alkylethersulfate (Verbindung a) der Formel (I) entsprechen:
**R¹(OCH₂CHR²)ₙOSO₃X** **(I)**
in der R¹ eine geradkettige oder verzweigte Alkyl- und/oder Alkenylgruppe mit 12 bis 18 Kohlenstoffatomen bedeutet, R² Wasserstoff oder Methyl bedeutet, n eine Zahl 1 bis 8 bedeutet und X Alkali, Erdalkali, Ammonium, Alkylammonium, Alkanolammonium oder Glucammonium bedeutet.

3. Zusammensetzungen nach den Ansprüchen 1 und/oder 2, **dadurch gekennzeichnet, daß** es sich bei der N-Acylaminosäure (Verbindung b1) um N-Cocoylglutamat handelt.

4. Zusammensetzungen nach einem der Ansprüche 1 bis 3, **dadurch gekennzeichnet, daß** die Alk(en)yloligoglycosidcarboxylate (Verbindung b2) der Formel (III) entsprechen:
**R⁴O[G]ₚO[(CH₂)ₘCOO⁻X⁺)ₙ** **(III)**
in der R⁴ einen Alkyl- oder Alkenylrest mit 6 bis 22 Kohlenstoffatomen bedeutet, G eine Zuckereinheit mit 5 oder 6 Kohlenstoffatomen bedeutet, p eine Zahl von 1 bis 10 bedeutet, m und n Zahlen von 1 bis 5 bedeuten und X Alkali, Erdalkali, Ammonium, Alkylammonium, Alkanolammonium oder Glucammonium bedeutet.

5. Zusammensetzungen nach einem der Ansprüche 1 bis 4, **dadurch gekennzeichnet, daß** die Alk(en)yloligoglycoside (Verbindung c) der Formel (IV) entsprechen:
**R⁵O[G]ₚ** **(IV)**
in der R⁵ einen Alkyl- oder Alkenylrest mit 4 bis 22 Kohlenstoffatomen bedeutet, G eine Zuckereinheit mit 5 oder 6 Kohlenstoffatomen bedeutet und p eine Zahl von 1 bis 10 bedeutet.

6. Zusammensetzungen nach einem der Ansprüche 1 bis 5, **dadurch gekennzeichnet, daß** die Partialglyceride der allgemeinen Formel (V) entsprechen: 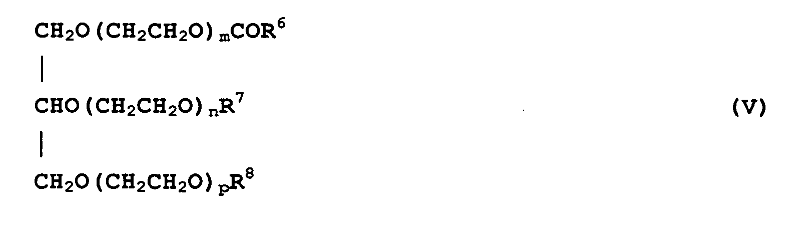 in der R⁶CO eine geradkettige oder verzweigte, gesättigte und/oder ungesättigte Acylgruppe mit 6 bis 22 Kohlenstoffatomen bedeutet, R⁷ und R⁸ unabhängig voneinander die gleiche Bedeutung wie R⁶CO aufweisen oder einen Hydroxylrest bedeuten und die Summe (m+n+p) 0 oder eine Zahl von 1 bis 100 ist, mit der Maßgabe, daß es sich bei mindestens einem der zwei Substituenten R⁷ und R⁸ um einen Hydroxylrest handelt.

7. Zusammensetzungen nach einem der Ansprüche 1 bis 6, **dadurch gekennzeichnet, daß** die amphoteren und/oder zwitterionischen Tenside (Verbindung e) aus der Gruppe der Alkylbetaine, Alkylamidobetaine, Imidazoline und Amphoglycinate stammen.

8. Zusammensetzungen nach Anspruch 7, **dadurch gekennzeichnet, daß** die amphoteren und/oder zwitterionischen Tenside der Formel (VI) entsprechen: 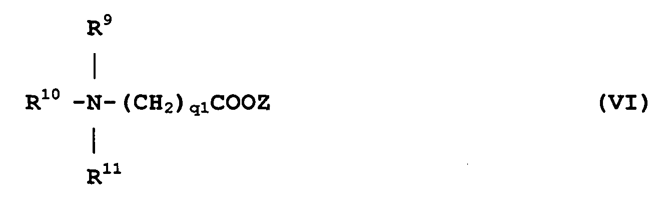 in der R⁹ einen Alkylrest mit 6 bis 22 Kohlenstoffatomen bedeutet, R¹⁰ Wasserstoff oder eine Alkylgruppe, die 1 bis 4 Kohlenstoffatome enthält, bedeutet, R¹¹ eine Alkylgruppe, die 1 bis 4 Kohlenstoffatome enthält, bedeutet, q1 eine Zahl von 1 bis 6 bedeutet und X ein Alkali- und/oder Erdalkalimetall oder Ammonium bedeutet.

9. Zusammensetzungen nach Anspruch 7, **dadurch gekennzeichnet, daß** die amphoteren und/oder zwitterionischen Tenside der Formel (VII) entsprechen: 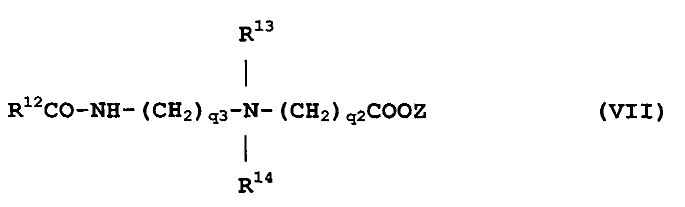 in der R¹²CO einen aliphatischen Acylrest mit 6 bis 22 Kohlenstoffatomen und 0 oder 1 bis 3 Doppelbindungen bedeutet, R¹³ Wasserstoff oder einen Alkylrest mit 1 bis 4 Kohlenstoffatomen bedeutet, R¹⁴ einen Alkylrest mit 1 bis 4 Kohlenstoffatomen bedeutet, q2 eine Zahl von 1 bis 6 bedeutet, q3 eine Zahl von 1 bis 3 bedeutet und Z ein Alkali- und/oder Erdalkalimetall oder Ammonium bedeutet.

10. Zusammensetzungen nach einem der Ansprüche 1 bis 9, **dadurch gekennzeichnet, daß** den kationischen Proteinen (Verbindung f) Abbauprodukte von Erbsen-, Reis-, Mandel-, Kartoffel-, Soja-, Seiden- und insbesondere Weizenproteinen zugrundeliegen.

11. Zusammensetzungen nach einem der Ansprüche 1 bis 10, **dadurch gekennzeichnet, daß** sie eine Viskosität von 1000 bis 4000 mPas aufweisen.

12. Verwendung von Mischungen enthaltend
(a) 1 bis 9 Gew.-% Alkylethersulfate,
(b) 0,1 bis 3 Gew.-% N-Acylaminosäuren und/oder Alk(en)yloligoglycosidcarboxylate,
(c) 0,1 bis 5 Gew.-% Alk(en)yloligoglycoside,
(d) 0,1 bis 2 Gew.-% Partialglyceride sowie gegebenenfalls
(e) 0,1 bis 5 Gew.-% amphotere und/oder zwitterionische Tenside und/oder
(f) 0,1 bis 1 Gew.-% kationische Proteine,
unter der Bedingung, daß die Komponenten mit Wasser 100 Gew.-% ausmachen für die Herstellung von Kosmetikzusammensetzungen.

## Revendications

1. Compositions nettoyantes douces, comprenant
(a) entre 1 et 9 % en masse de sulfates d'alkyléther,
(b) entre 0,1 et 3 % en masse d'acides N-acylaminés et/ou de carboxylates d'oligoglycosides d'alkyle ou d'alcényle,
(c) entre 0,1 et 5 % en masse d'oligoglycosides d'alkyle ou d'alcényle,
(d) entre 0,1 et 2 % en masse de glycérides partiels, et éventuellement
(e) entre 0,1 et 5 % en masse de tensioactifs amphotères et/ou zwitterioniques et/ou
(f) entre 0,1 et 1 % en masse de protéines cationiques
à la condition que la quantité totale des composés ajoutés à de l'eau et éventuellement à d'autres substances auxiliaires typiques soit égale à 100 % en masse.

2. Compositions selon la revendication 1, **caractérisées en ce que** lesdits sulfates d'alkyléther (composé a) répondent à la formule (I) :
**R¹(OCH₂CHR²)ₙOSO₃X** **(I)**
où R¹ représente un groupement alkyle et/ou alcényle linéaire ou ramifié contenant entre 12 et 18 atomes de carbone, R² représente un atome d'hydrogène ou un groupement méthyle, n est un nombre compris entre 1 et 8 et X représente un métal alcalin ou alcalino-terreux ou un groupement ammonium, alkylammonium, alcanolammonium ou glucammonium.

3. Compositions selon les revendications 1 et/ou 2, **caractérisées en ce que** ledit acide N-acylaminé (composé b1) est le glutamate de N-cocoyle.

4. Compositions selon l'une quelconque des revendications 1 à 3, **caractérisées en ce que** lesdits carboxylates d'oligoglycosides d'alkyle ou d'alcényle (composé b2) répondent à la formule (III) :
**R⁴O[G]ₚO[(CH₂)ₘCOO⁻X**⁺]ₙ **(III)**
où R⁴ représente un radical alkyle ou alcényle comportant entre 6 et 22 atomes de carbone, G est un motif sucre comportant 5 ou 6 atomes de carbone, p est un nombre compris entre 1 et 10, m et n sont des nombres compris entre 1 et 5 et X représente un métal alcalin ou alcalino-terreux ou un groupement ammonium, alkylammonium, alcanolammonium ou glucammonium.

5. Compositions selon l'une quelconque des revendications 1 à 4, **caractérisées en ce que** lesdits oligoglycosides d'alkyle ou d'alcényle (composé c) répondent à la formule (IV) :
**R⁵O[G]ₚ** **(IV)**
où R⁵ représente un radical alkyle ou alcényle comportant entre 4 et 22 atomes de carbone, G représente un motif sucre comportant 5 ou 6 atomes de carbone et p est un nombre compris entre 1 et 10.

6. Compositions selon l'une quelconque des revendications 1 à 5, **caractérisées en ce que** lesdits glycérides partiels répondent à la formule générale (V), 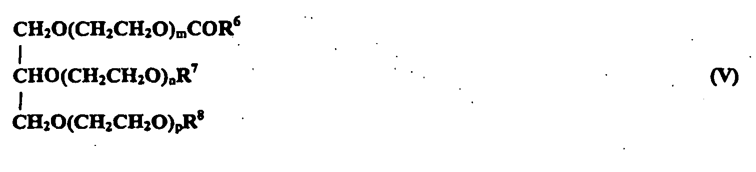 où R⁶CO représente un groupement acyle linéaire ou ramifié, saturé et/ou insaturé comportant entre 6 et 22 atomes de carbone, R⁷ et R⁸, indépendamment l'un de l'autre, prennent l'une des valeurs de R⁶CO ou représentent un radical hydroxyle, et la somme (m+n+p) est égale à 0 ou à un nombre compris entre 1 et 100, à la condition qu'au moins un des deux substituants R⁷ et R⁸ représente un radical hydroxyle.

7. Compositions selon l'une quelconque des revendications 1 à 6, **caractérisées en ce que** lesdits tensioactifs amphotères et/ou zwitterioniques (composé e) sont sélectionnés au sein du groupe constitué par les alkylbétaïnes, les alkylamidobétaïnes, les imidazolines et les amphoglycinates.

8. Compositions selon la revendication 7, **caractérisées en ce que** lesdits tensioactifs amphotères et/ou zwitterioniques répondent à la formule (VI) : 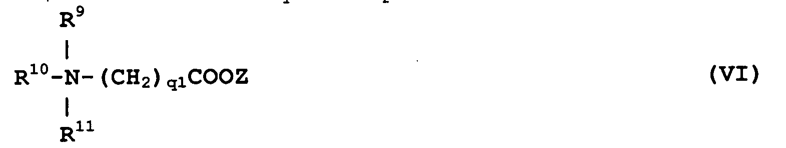 où R⁹ représente un radical alkyle comportant entre 6 et 22 atomes de carbone, R¹⁰ représente un atome d'hydrogène ou un groupement alkyle comportant entre 1 et 4 atomes de carbone, R¹¹ représente un groupement alkyle comportant entre 1 et 4 atomes de carbone, q1 est un nombre compris entre 1 et 6 et X représente un métal alcalin et/ou alcalino-terreux ou un groupement ammonium.

9. Compositions selon la revendication 7, **caractérisées en ce que** lesdits tensioactifs amphotères et/ou zwitterioniques répondent à la formule (VII) : 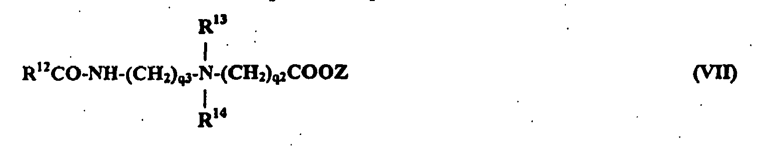 où R¹²CO représente un radical acyle aliphatique comportant entre 6 et 22 atomes de carbone et 0 ou entre 1 et 3 doubles liaisons, R¹³ représente un atome d'hydrogène ou un radical alkyle comportant entre 1 et 4 atomes de carbone, R¹⁴ représente un radical alkyle comportant entre 1 et 4 atomes de carbone, q² représente un nombre compris entre 1 et 6, q³ représente un nombre compris entre 1 et 3 et Z représente un métal alcalin et/ou alcalino-terreux ou un groupement ammonium.

10. Compositions selon l'une quelconque des revendications 1 à 9, **caractérisées en ce que** lesdites protéines cationiques (composé f) sont basées sur des produits de dégradation de petits pois, de riz, d'amandes, de pommes de terre, de soja, de soie et en particulier de protéines de blé.

11. Compositions selon l'une quelconque des revendications 1 à 10, **caractérisées en ce qu'**elles présentent une viscosité comprise entre 1000 et 4000 mPa.

12. Emploi de mélanges comprenant
(a) entre 1 et 9 % en masse de sulfates d'alkyléther,
(b) entre 0,1 et 3 % en masse d'acides N-acylaminés et/ou de carboxylates d'oligoglycosides d'alkyle ou d'alcényle,
(c) entre 0,1 et 5 % en masse d'oligoglycosides d'alkyle ou d'alcényle,
(d) entre 0,1 et 2 % en masse de glycérides partiels, et éventuellement
(e) entre 0,1 et 5 % en masse de tensioactifs amphotères et/ou zwitterioniques et/ou
(f) entre 0,1 et 1 % en masse de protéines cationiques à la condition que la quantité totale des composés ajoutés à de l'eau soit égale à 100 % en masse, dans la fabrication de compositions cosmétiques.
